# EUROPÄISCHE PATENTSCHRIFT

(11) **EP 0 754 666 B1**
(45) Veröffentlichungstag und Bekanntmachung des Hinweises auf die Patenterteilung: **06.10.1999**
(21) Anmeldenummer: 96110886.7
(22) Anmeldetag: 05.07.1996
(51) Int. Cl.: C07C 37/20

(54) **Verfahren zur Herstellung von Bis(4-hydroxyaryl)alkanen**
Process for the preparation of bis-(4-hydroxyaryl)alcanes
Procédé de préparation de bis(4-hydroxyaryl)alcanes

(30) Priorität: 18.07.1995 DE 19526088
(43) Veröffentlichungstag der Anmeldung: 22.01.1997
(73) Patentinhaber: BAYER AG, 51368 Leverkusen (DE)
(72) Erfinder: Berg, Klaus, Dr., 47798 Krefeld (DE); Buysch, Hans-Josef, Dr., 47809 Krefeld (DE); Eitel, Alfred, Dr., 41539 Dormagen (DE); Fennhoff, Gerhard, Dr., 47877 Willich (DE); Malamet, Georg, Dr., 47800 Krefeld (DE); Wulff, Clauss, Dr., 47800 Krefeld (DE)

(56) Entgegenhaltungen:
- EP-A- 0 342 758
- EP-A- 0 616 993
- GB-A- 785 079
- US-A- 4 301 305
- US-A- 4 391 997
- US-A- 4 400 555

## Beschreibung

Die vorliegende Erfindung betrifft ein Verfahren zur Herstellung von Bis(4-hydroxyaryl)alkanen durch heterogen säurekatalysierte Umsetzung von aromatischen Hydroxyverbindungen mit Ketonen in in Reihe geschalteten Reaktoren, die in Richtung fortschreitender Umsetzung mit steigender Temperatur und gegebenenfalls mit steigender Belastung betrieben werden.

Es ist bekannt, in einem Prozeß zur Herstellung von Bis(4-hydroxyaryl)alkanen aus Phenolen und Ketonen die erforderliche Ketonmenge auf mehrere in Reihe geschaltete Reaktoren zu verteilen. Die US-PS 2 775 620 beschreibt ein mit Mineralsäure katalysiertes Verfahren in homogener flüssiger Phase; ein heterogen katalysiertes Verfahren mit einem sauren Ionenaustauscher in einem Festbettreaktor geht aus US-PS 4 400 555 (EP-A 342 758) hervor. Beide Schriften belegen, daß durch Aufteilung der Ketonmenge der Anteil an Nebenprodukten reduziert wird, wobei dieser Anteil bei HCl-Katalyse geringer ist als bei Katalyse mit Ionentauschern. Allerdings wird dies nur für ein Eduktgemisch aus Phenol und Aceton demonstriert. Bei einer kontinuierlich betriebenen Produktionsanlage wird jedoch in der Regel die nach Abtrennung des Bis(4-hydroxyaryl)alkans aus dem Reaktionsgemisch erhaltene Mutterlauge wieder in den Prozeß zurückgeführt. Dabei können sich Isomeren und Nebenprodukte anreichern, was zu Störungen im Prozeß führt, z.B. schlechterer Kristallisation des Bis(4-hydroxyaryl)alkans und geringerer Produktqualität.

Daher ist es wunschenswert, Verfahren zu entwickeln, bei denen unter den Bedingungen einer kontinuierlich betriebenen Anlage möglichst geringe Anteile an Isomeren und Nebenprodukten gebildet werden.

Es wurde nun ein Verfahren gefunden zur Herstellung von Bis(4-hydroxyaryl)alkanen durch heterogen sauer katalysierte Umsetzung aromatischer Hydroxyverbindungen mit Ketonen in mindestens zwei in Reihe geschalteteten Festbettreaktoren, die in Richtung fortschreitender Umsetzung mit steigenden Temperaturen und steigenden Belastung betrieben werden, wobei die Gesamtmenge an Keton auf die einzelnen Reaktoren aufgeteilt und vor dem Eintreten in die jeweiligen Katalysatorbetten homogen im Reaktionsgemisch verteilt wird.

Geeignete aromatische Hydroxyverbindungen für das erfindungsgemäße Verfahren sind in p-Position nicht substituiert und enthalten keine Substituenten zweiter Ordnung wie Cyano-, Carboxy- oder Nitrogruppen; genannt seien beispielsweise Phenol, o- und m-Kresol, 2,6-Dimethylphenol, o-tert.-Butylphenol, 2-Methyl-6-tert.-Butylphenol, o-Cyclohexylphenol, o-Phenylphenol, o-Isopropylphenol, 2-Methyl-6-cyclopentyl-phenol, o- und m-Chlorphenol, 2,3,6-Trimethylphenol. Bevorzugt sind Phenol, o- und m-Kresol, 2,6-Dimethylphenol, o-tert.-Butylphenol und o-Phenyl-phenol; besonders bevorzugt ist Phenol.

Geeignete Ketone enthalten wenigstens eine aliphatische Gruppe an der Carbonylfunktion; genannt seien beispielsweise Aceton, Methylethylketon, Methylpropylketon, Methylisopropylketon, Diethylketon, Acetophenon, Cyclo-hexanon, Cyclopentanon, Methyl-, Dimethyl- und Trimethylcyclohexanone, die auch geminale Methylgruppen aufweisen können wie 3,3-Dimethyl-5-methylcyclohexanon (Hydroisophoron). Bevorzugt sind Aceton, Acetophenon, Cyclohexanon und dessen Methylgruppen tragende Homologe; besonders bevorzugt ist Aceton.

Bevorzugte Edukte für das erfindungsgemäße Verfahren sind insbesondere auch die nach Abtrennung der Bis(4-hydroxyaryl)alkane verbleibenden Mutterlaugen, die nach Zugabe der verbrauchten Hydroxyverbindungen und gegebenenfalls Entnahme eines gewissen Anteils zur Vermeidung der Anreicherung unerwünschter Beiprodukte in das Verfahren zurückgeführt werden; für den Fall der Synthese von Bisphenol A enthalten solche Mutterlaugen ca. 78-88 Gew.-% Phenol und 12-22 Gew.-% Bisphenol A und Beiprodukte, die sich wie folgt zusammensetzen:

| | |
|---|---|
| Bisphenol A | 40-65 Gew.-% |
| o,p-Bisphenol | 14-19 Gew.-% |
| Trisphenol | 2-6 Gew.-% |
| Chromane | 4-17 Gew.-% |
| 1,3,3-Trimethyldihydroxyphenylindane | 3-13 Gew.-% |
| weitere Nebenprodukte | 3-15 Gew.-% |

Das Molverhältnis von aromatischer Hydroxyverbindung zu Keton beträgt im allgemeinen 5:1 bis 25:1, bevorzugt 7:1 bis 20:1, besonders bevorzugt 8:1 bis 18:1, bezogen auf die Gesamtreaktion.

Das eingesetzte Eduktgemisch kann geringe Mengen Wasser enthalten, bevorzugt weniger als 1, besonders bevorzugt weniger als 0,6 und ganz besonders bevorzugt weniger als 0,3 Gew.-%.

Die als Katalysatoren eingesetzten Ionentauscherharze und die als Cokatalysatoren verwendeten Mercaptoverbindungen sind dem Fachmann bekannt (US-A 2 468 982; 2 623 908; 2 775 620; DE-OS 3 619 450; 3 727 641).

Im kontinuierlichen Betrieb wird vor jedem Reaktionszyklus dem Reaktionsgemisch die im vorhergehenden Zyklus verbrauchte Menge an aromatischer Hydroxyverbindung wieder zugesetzt. Die zur Einstellung des gewünschten Molverhältnisses von Hydroxyverbindung und Keton benötigte Menge an Keton wird auf die n Reaktoren der Produktionsanlage aufgeteilt, wobei man dem Reaktionsgemisch vor jedem Reaktor etwa den n-ten Teil der gesamten Ketonmenge zudosiert. Die Abweichung von diesem Wert kann für den einzelnen Reaktor absolut ± 15 %, bevorzugt ± 10 %, besonders bevorzugt ± 5 % betragen.

Die Anzahl der Reaktoren beträgt mindestens zwei und wird aus Gründen der Wirtschaftlichkeit in der Regel nicht mehr als acht, bevorzugt nicht mehr als sechs, besonders bevorzugt nicht mehr als vier betragen.

Die Belastung, definiert als Menge (in kg) Eduktgemisch pro Liter Katalysator im Betriebszustand (gequollen) und pro Stunde liegt pro Reaktor bei 0,1 bis 2,0, bevorzugt 0,15 bis 1,7, besonders bevorzugt 0,19 bis 1,5 kg / l · h. Die Belastung sollte in der Regel so gewählt werden, daß der Umsatz an Aceton nach dem letzten Reaktor mindestens 75%, besser SSge$$ 83%, bevorzugt ≥ 90% und besonders bevorzugt ≥ 95% beträgt.

Es ist erforderlich, alle Reaktoren mit steigenden Belastung zu betreiben. mit 0,3, der zweite Reaktor mit 0,6 und der dritte Reaktor mit 0,8 kg / l · h betrieben werden.

Für eine wirksame Verminderung der Beiproduktmenge ist es sehr wichtig, daß vor dem Eintritt in das jeweilige Katalysatorbett das Keton völlig homogen im Reaktionsgemisch verteilt wird, was durch den Einsatz von Düsen, statischen Mischern, Rührbehältern, Kreiselpumpen oder anderen dem Fachmann geläufigen Mischapparaturen erreicht werden kann.

Die in Reihe geschalteten Reaktoren werden in Richtung fortschreitender Umsetzung mit steigenden Temperaturen betrieben. Zwischen Anfang und Ende der Reaktorkaskade wird ein ansteigendes Temperaturprofil im Temperaturbereich von 40 bis 100°C, vorzugsweise 45 bis 90°C, besonders bevorzugt 50 bis 85°C eingestellt. Die Temperaturunterschiede von einem zum nächsten Reaktor werden in der Regel umso geringer sein, je mehr Reaktoren durchlaufen werden sollen. Es ist auch möglich, zwei aufeinanderfolgende Reaktoren mit gleicher Temperatur zu betreiben.

Da in Festbettreaktoren keine Durchmischung stattfindet und die Abführung der Reaktionswärme aus dem Reaktionsgemisch schwierig ist, werden solche Reaktoren in der Regel adiabatisch betrieben, was zur Erwärmung des Reaktionsgemischs führt. Es ist daher meist zweckmäßig, das Reaktionsgemisch zwischen den einzelnen Reaktoren zu kühlen, wobei darauf zu achten ist, daß ein Auskristallieren von Bis(4-hydroxyaryl)alkan, das ein Verstopfen der Rohrleitungen zur Folge hätte, vermieden wird.

Im folgenden soll die Erfindung beispielhaft erläutert werden, wobei nur Beispiel 10 als erfindungsgemäß zu betrachten ist.

### Beispiel 1

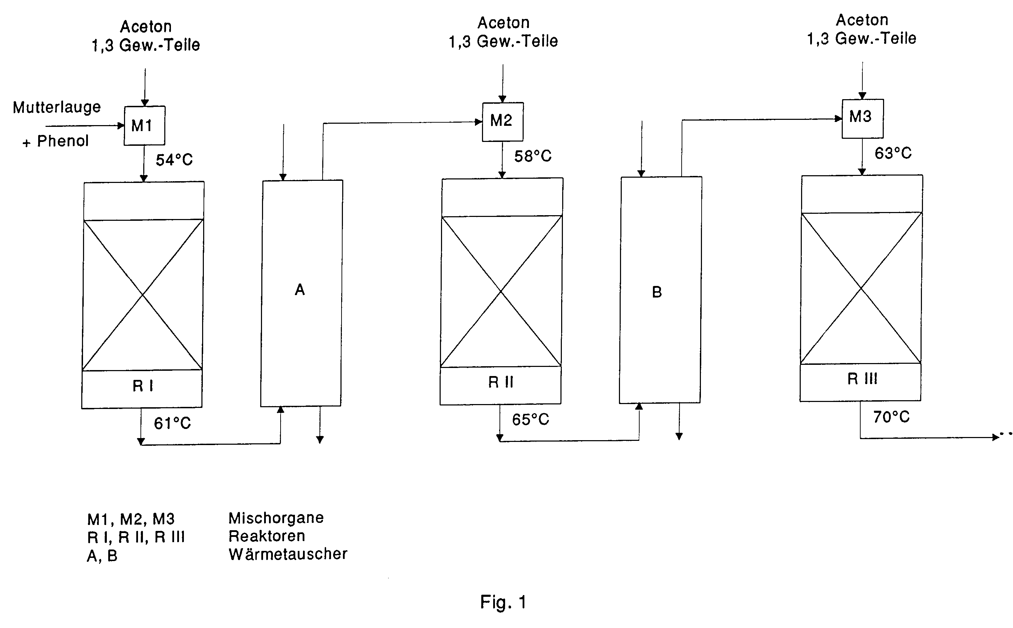

Der Versuchsaufbau ist in Figur 1 schematisch wiedergegeben. Die angegebenen Zahlenwerte sind beispielhaft zu verstehen. In den ersten einer Serie von drei hintereinander geschalteten, mit einem mit 2 % Divinylbenzol vernetzten und mit 5 % Cysteamin beladenen sulfonierten Polystyrolharz gefüllten Festbettreaktoren wurde eine Mischung aus 98,7 Gewichtsteilen einer Mutterlauge aus laufender Bisphenol A-Produktion, der verbrauchtes Phenol wieder zugesetzt war, und 1,3 Gewichtsteilen Aceton, die in einem Gefäß gut gemischt worden waren, mit einer Durchschnittstemperatur von 61°C und einer Belastung von 0,2 kg / l · h unter N₂ durch das Katalysatorbett geschickt.

Das den Reaktor verlassende Reaktionsgemisch wurde gesammelt, wieder mit 1,3 Gewichtsteilen Aceton gut vermischt und mit einer Durchschnittstemperatur von 66°C und einer Belastung von 0,2 kg / l · h durch das zweite Reaktorbett geleitet.

Dein aus diesem Reaktor abfließenden Produktgemisch wurden wiederum 1,3 Gewichtsteile Aceton zugemischt und die Mischung mit einer Durchschnittstemperatur von 71°C und einer Belastung von 0,2 kg / l · h durch den dritten Reaktor geleitet.

Der Acetonumsatz war vollständig. Der Versuch wurde über 535 h durchgeführt. Die durch tägliche Analysen erhaltene gemittelte Zusammensetzung des Reaktionsprodukts nach dem dritten Reaktor zeigt Tabelle 1.

### Beispiel 2

In einem Versuch analog Beispiel 1 wurde die Gesamtmenge von 3,9 Gewichtsteilen Aceton dem Reaktionsgemisch vor Reaktor I zugemischt. Die Reaktionsmischung wurde mit einer Belasung von 0,2 kg / l · h aufgegeben und bei einer Durchschnittstemperatur von 70°C umgesetzt.

Der Acetonumsatz war vollständig. Der Versuch wurde über 468 h durchgeführt. Die durch tägliche Analysen erhaltene gemittelte Zusammensetzung des Reaktionsprodukts nach dem Reaktor zeigt Tabelle 1.

### Beispiel 3

Bei Durchführung des Versuchs analog Beispiel 2 bei einer Durchschnittstemperatur von 65°C kristallisierte das Reaktionsgemisch im Reaktor aus. Der Versuch mußte abgebrochen werden.

**Tabelle 1**

| | o,p-BP | BPA | Trisphenol | Chromane | restl. NP |
|---|---|---|---|---|---|
| Edukt in 85 Teilen Phenol | 17,74 | 56,31 | 4,37 | 6,31 | 15,27 |
| Beispiel 1 | 8,10 | 80,40 | 1,83 | 2,76 | 6,91 |
| Beispiel 2 | 9,80 | 78,40 | 2,22 | 3,16 | 6,42 |
| Beispiel 3 | Wegen Kristallisation im Reaktor abgebrochen | | | | |

Die Versuche zeigen, daß die Selektivität für Bisphenol A (BPA) im kontinuierlichen Betrieb durch geteilte Acetondosierung, homogene Verteilung des Acetons in der umzusetzenden Reaktionsmischung und steigender Temperatur entlang der Reaktionsstrecke in Richtung fortschreitender Umsetzung um 2% erhöht wird.

### Beispiel 4

Beipiel 1 wurde mit einer Mutterlauge aus laufender BPA-Produktion wiederholt, die eine andere Zusammensetzung aufwies als in Beispiel 1. Die Zusammensetzung des Edukts und der Produktmischung sind in Tabelle 2 aufgeführt.

### Beispiel 5

Beispiel 2 wurde wiederholt mit der in Beispiel 4 verwendeten Mutterlauge. Die Zusammensetzung des Reaktionsgemischs ist in Tabelle 2 aufgeführt.

### Beispiel 6

Es wurde ein Versuch analog Beispiel 4 durchgeführt, bei dem alle Reaktoren bei einer Durchschnittstemperatur von 70°C betrieben wurden. Die Zusammensetzung des Reaktionsgemischs ist in Tabelle 2 aufgeführt.

**Tabelle 2**

| | o,p-BP | BPA | Trisphenol | Chromane | restl. NP |
|---|---|---|---|---|---|
| Edukt in 85 Teilen Phenol | 16,75 | 58,19 | 3,75 | 7,80 | 13,46 |
| Beispiel 4 | 7,95 | 79,25 | 1,82 | 3,80 | 7,18 |
| Beispiel 5 | 9,19 | 77,26 | 2,14 | 4,00 | 7,41 |
| Beispiel 6 | 7,23 | 78,20 | 1,80 | 4,56 | 8,21 |

Die Versuchsdauer in Beispiel 4 bis 6 betrug jeweils 250 bis 270 h. Es zeigt sich, daß die Selektivitätserhöhung trotz geänderter Zusammensetzung wiederum ca. 2% beträgt. Im Vergleich von Beispiel 4 und Beispiel 6 wird der Effekt des Temperaturgradienten auf die Selektivität deutlich.

### Beispiel 7

Beispiel 1 wurde mit einer Mutterlauge aus laufender BPA-Produktion wiederholt, die eine andere Zusammensetzung aufwies als in Beispiel 1. Der Acetonumsatz war vollständig; die Versuchsdauer betrug 108 h. Die Zusammensetzung der Edukt- und der Produktmischung sind in Tabelle 3 aufgeführt.

### Beispiel 8

Beispiel 7 wurde mit einer Belastung von 0,4 kg / l · h wiederholt. Der Acetonumsatz war vollständig; die Versuchsdauer betrug 80 h. Die Zusammensetzung der Produktmischung ist in Tabelle 3 aufgeführt.

### Beispiel 9

Beispiel 7 wurde mit einer Belastung von 0,6 kg / l · h wiederholt. Der Acetonumsatz lag über 98%; die Versuchsdauer betrug 98 h. Die Zusammensetzung der Produktmischung ist in Tabelle 3 aufgeführt.

### Beispiel 10

Es wurde ein Versuch analog Beispiel 7 durchgeführt, wobei Reaktor I mit einer Belastung von 0,2 kg / l · h, Reaktor II mit einer Belastung von 0,4 kg / l · h und Reaktor III mit einer Belastung von 0,6 kg / l · h betrieben wurden. Der Acetonumsatz war > 98%; die Versuchsdauer betrug 82 h. Die Zusammensetzung der Produktmischung ist in Tabelle 3 aufgeführt.

**Tabelle 3**

| | o,p-BP | BPA | Trisphenol | Chromane | restl. NP |
|---|---|---|---|---|---|
| Edukt in 85 Teilen Phenol | 16,69 | 59,29 | 3,60 | 8,09 | 12,33 |
| Beispiel 7 | 7,97 | 79,77 | 1,81 | 3,95 | 6,50 |
| Beispiel 8 | 8,20 | 79,79 | 1,85 | 3,90 | 6,26 |
| Beispiel 9 | 8,48 | 79,46 | 1,95 | 3,87 | 6,24 |
| Beispiel 10 | 8,18 | 79,93 | 1,83 | 3,85 | 6,21 |

Es zeigt sich, daß die Selektivität auch bei hoher Belastung erhalten bleibt und sich durch Anlegen eines Belastungsgradienten in Richtung fortschreitender Umsetzung weiter steigern läßt.

### Beispiel 11

Versuch 1 wurde wiederholt; in der Eduktmischung wurde anstelle einer Mutterlauge aus der BPA-Produktion reines Phenol verwendet. Die Zusammensetzung des Produktgemischs nach dem dritten Reaktor zeigt Tabelle 4.

### Beispiel 12

Versuch 2 wurde wiederholt; in der Eduktmischung wurde anstelle einer Mutterlauge aus der BPA-Produktion reines Phenol verwendet. Die Zusammensetzung des Produktgemischs nach dem dritten Reaktor zeigt Tabelle 4.

**Tabelle 4**

| | o,p-BP | BPA | Trisphenol | Chromane | restl. NP |
|---|---|---|---|---|---|
| Beispiel 11 | 5,68 | 93,50 | 0,47 | 0,12 | 0,23 |
| Beispiel 12 | 6,76 | 91,90 | 0,48 | 0,46 | 0,40 |

Auch bei Verwendung von reinem Phenol anstelle einer Mutterlauge aus der BPA-Produktion läßt sich der Anteil an Bisphenol A im Produktgemisch durch homogene Verteilung der Acetongaben im Reaktionsgemisch und das Anlegen eines Temperaturgradienten in Richtung fortschreitender Umsetzung steigern.

## Patentansprüche

1. Verfahren zur Herstellung von Bis(4-hydroxyaryl)alkanen durch heterogen sauer katalysierte Umsetzung aromatischer Hydroxyverbindungen mit Ketonen in mindestens zwei in Reihe geschalteten Festbettreaktoren, die in Richtung fortschreitender Umsetzung mit steigenden Temperaturen und steigender Belastung betrieben werden, wobei die Gesamtmenge an Keton auf die einzelnen Reaktoren aufgeteilt und vor dem Eintreten in die jeweiligen Katalysatorbetten homogen im Reaktionsgemisch verteilt wird.

## Claims

1. Process for the preparation of bis(4-hydroxyaryl)alkanes by the reaction, under heterogeneous acid catalysis, of aromatic hydroxyl compounds with ketones in at least two fixed bed reactors connected in series, which are operated in such a way that the reaction progresses with increasing temperatures and increasing load, wherein the total amount of ketone is divided up among the individual reactors and homogeneously distributed in the reaction mixture before entering the respective catalyst beds.

## Revendications

1. Procédé pour la préparation de bis(4-hydroxyaryl)alcanes par réaction hétérogène catalysée par un acide de composés hydroxy aromatiques avec des cétones dans au moins deux réacteurs en lit fixe raccordés en série, lesquels fonctionnent dans la direction de l'avancement de la réaction avec des températures croissantes et une charge croissante, la quantité totale de cétone étant répartie sur les réacteurs individuels et étant distribuée avant l'entrée dans les lits de catalyseur respectifs de manière homogène dans le mélange réactionnel.
